# EUROPEAN PATENT APPLICATION

(11) **EP 1 577 281 A1**
(43) Date of publication of application: **21.09.2005**
(21) Application number: 04006669.8
(22) Date of filing: 19.03.2004
(51) Int. Cl.: C07B 61/00, H05B 6/64, B01L 7/00

(54) **Process for preparing combinatorial libraries**

(71) Applicant: Bayer CropScience GmbH, 65929 Frankfurt/Main (DE)
(72) Inventor: Lange, Tim, 15133 Södertälje (SE); Lindell, Stephen, 65779 Kelkheim-Fischbach (DE)

(57) **Abstract**

A process for the production of a combinatorial library of where a plurality of organic molecules is synthesized by chemically fixing a plurality of starting materials each to a separate portion of solid resin, which is combined with a radiofrequency tag, building up the organic molecules in multiple reaction steps, and finally cleaving of the desired organic molecules, where at least one of the multiple reaction steps is carried out under microwave irradiation.

## Description

The invention relates to a process for preparing combinatorial libraries using solid supports and radiofrequency readable ("rf") tags, to the use of microwave irradiation in such a process and to a device for carrying out the process.

Combinatorial chemistry performed on a solid phase support has become a widespread technique, particularly in the discovery of biologically active compounds, for the generation of large number of molecules, called compound libraries, to be subsequently submitted to a biological screening process (see, e.g. K. M. Jenkins, R. Angeles, M. T. Quintos, R. Xu, D. B. Kassel, R. A. Rourick, *J. Pharmaceutical and Biomedical Analysis* 2004, 34(5), 989-1004; WO-A 04/14929).

The solid phase, an example being a polystyrene matrix in a small bead like shape, that macroscopically appears like a powder, has an anchor group, to which the molecule, that is being built up in the process of the library synthesis, is attached. This material is often titled "resin". At the end of the combinatorial synthesis, the molecules generated are cleaved off the resin.

There are two strategies for synthesizing libraries of compounds on a solid phase support: Either resin portions are held specially separate or they are pooled as mixtures.

If resin portions are held specially separate, it is advantageous in the combinatorial synthesis process to use the "split and mix" method (see, e.g. WO-A 98/15532). Portions of the resin are put into a set of capsules that can contain the resin, but are permissible for dissolved reagents. Alternatively, the resin portion can be formed into a solid object, e.g. a cylinder or ball. The resin portions are being separated to undergo different chemistry ("split"), then combined again ("mix"), before being split again for the next reaction sequence. In order to follow, what chemistry a resin portion has been exposed to and hence, what final molecule was synthesized, the use of rf-tags has been reported, that accompany the resin portion throughout all manipulations. This is possible either by incorporating them together with the resin into capsules, or if the resin portion form a solid object the rf-tag can be embedded into it. For example, the resin portion can be shaped like a cylinder having a center hole, into which the rf-tag is inserted.

This tag technology allows directing the manipulation of resin portions, e.g. contained in capsules, due to online computer aided scanning of the radiofrequency tags. The rf-tags contain three components: first a memory for alphanumeric codes; second a rectifying circuit which absorbs radiofrequency energy and converts this energy into electrical energy. The latter is used by the third component, an antenna, to transmit the code to an external receiver that is linked to a computer. The computer then links the signal to the chemical history of the resin portion. There are capsules that are commercialized under the brand name MacroKan®, MiniKan® or MicroKan® (Discovery Partners Intl., 9640 Towne Centre Drive, San Diego CA 92121, U.S.A.). Resin portions formed into sizeable solid objects are known under the brand name SynPhase Lanterns® (Mimotopes, Suite F, 4178 Sorrento Valley Boulevard, SAN DIEGO, CA 92121, U.S.A.) and Stratospheres Plugs® (Polymer Laboratories Inc., Amherst Fields Research Park, 160 Old Farm Road, Amherst MA 01002, U.S.A.). This process of using rf-tags to direct sorting of resin portions contained in Kans for the purpose of building up a combinatorial library is called the IRORI technology (see e.g. WO-A 96/24061; K. C. Nicolaou., X. Y. Xiao, Z. Parandoosh, A. Senyei, M. Nova, *Angew. Chem., Int. Ed. Engl.* 1995, 34, 2289-2291).

In comparison to the chemistry in liquid phase, the chemistry on solid phase suffers from the drawback of slow reaction rates and often reduced yield. To overcome this problem typically large excesses of reagents are applied. Alternatively, the temperature of a reaction can be increased to speed up the reaction. However, the nature of the polymeric matrix of the resin and the material of the resin containers or the nature of the micro titerplate can set limits. For example, above a certain temperature the polymeric matrix of the resin and/or the capsules can melt. Accordingly, there was the task to develop new technologies for solid phase combinatorial chemistry, in order to overcome, at least partly, the above mentioned drawbacks.

Surprisingly, it was now found that the application of microwave heating in solid phase combinatorial chemistry using rf-tag technology leads to significantly increased yields of products without causing material degradation of either resin, capsule material or rf-tag, when exposed to high thermal energy and electromagnetic fields in the form of microwaves.

This is surprising, because both the material of the capsule and the resin itself are polymers that have only a limited thermal stability, such that at higher temperatures they melt and lose their material properties.

Likewise, it was surprising to find, that it is also possible to apply microwave heating for the purpose of performing combinatorial chemistry to resin portions in the form of a solid object, like e.g. Lantern® or Plug®, into which a rf-tag was introduced, without observing material degradation to either the resin material or the rf-tag.

It was surprising, that the radiofrequency tag used for tracking of the resin portions during the synthesis and for directed sorting were still operable after extended exposure to microwaves. Thus, the exposure of the rf-tags to intense electromagnetic irradiation in the microwaves wavelength does not harm the three components of the tag. In addition, it was surprising, that the rf-tag does not absorb so much energy, that the surrounding polymeric material (resin and Kan, Lantern or Plug) was degraded, which would cause material property losses. This was unexpected, since the rf-tag material consists of conducting material that absorbs energy of microwave irradiation very well, as is known from the behavior of metallic material in microwave ovens.

In general, techniques have been already been developed to perform solid phase chemistry in combination with microwave heating (see. e.g. Combinatorial Chemistry and High Throughput Screening 2002, 5, 441-458). So far no technique has been described to combine the advantages of microwave heating for solid phase combinatorial chemistry with the technique of radiofrequency labeling of the resin holding container.

Accordingly, in one aspect of the invention there is provided a process for the production of a combinatorial library where a plurality of organic molecules is synthesized by chemically fixing a plurality of starting materials each to a separate portion of a solid support, which is combined with a radiofrequency tag, building up the organic molecules in multiple reaction steps, and finally cleaving off the desired organic molecules from the solid support, where at least one of the multiple reaction steps is carried out under microwave irradiation.

In a further aspect of the invention there is provided the use of microwave irradiation in the synthesis of combinatorial libraries on solid supports that are combined with radiofrequency tags.

In yet a further aspect of the invention there is provided a device for the production of a combinatorial library comprising a microwave oven with means for orbitally or linearly shaking vessels inside the microwave cavity. Thus a thorough mixing of the reaction partners in the vessel(s) during the reaction time is achieved. It is advantageous to have mixing by orbital or linear shaking, as the nature of the resin and/or the capsule may not withstand stirring - either magnetically or mechanically - very well. Due to this circumstance, the use of orbital or linear shaking is common practice in solid phase chemistry, yet so far no microwave oven has been described using this principle. Linear shaking can be achieved by eccentrically fixing the axle, that commonly drives rotating caroussels in microwave ovens, like e.g. the MS Ethos, or pizza plate holders in ordinary household microwaves, to a table holding the reaction vessels. One preferred way of realizing this setup is illustrated in

### Figure 1.

To the rotating axle protruding from the ground plate of the cubic interior of a microwave oven is fixed a disc holding eccentrically a pin. The pin fits into a groove on the bottom side of the table supporting the reaction vessels. As the axle rotates, the pin describes a circular movement that can be described as a movement with x and y vectors. Since the pin moves in a grove, only one vector component of the pin movement is translated into a movement of the supporting table, creating a translatory back-and-forth movement

The frequency of the vibration is then dependent of the rotating speed of the axle.

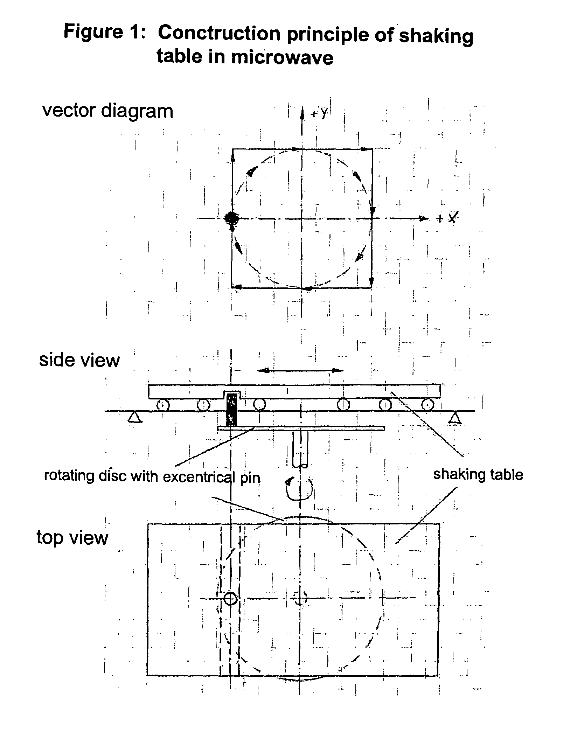

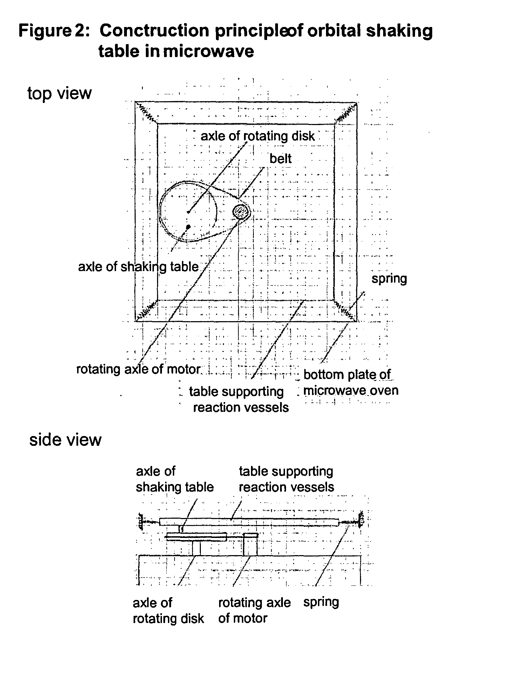

Alternative construction principles can similarly be employed to create orbital shaking movements of the table supporting the reaction vessels. The rotating axle protruding from the ground plate of the cubic interior of a microwave oven could translate its motion via a belt to a disk. This disk supports the table holding the reaction vessels. The support for the table is eccentrically positioned on that disk. As the disk rotates, it induces an orbital movement of the table, since springs attaching the table to the microwave cavity only allow the table to follow the movement of the rotating disk partially. This principle is illustrated in Figure 2.

The process according to the invention offers several advantages like increased reaction rate, reduction in reagent excesses and improved product purity. The increased reaction speed allows a reduction of the time of exposure to critical temperatures for both the resin and the organic molecules grafted to the resin.

The process often leads to improved yields and in some cases represents the only way of achieving the desired reaction.

The rf-tags used according to the invention include all common types. They are commercially available e.g. from Discovery Partners Intl., 9640 Towne Centre Drive, San Diego CA 92121, U.S.A.

Resins that can be used according to the invention are well known to the scientist skilled in the art of solid phase chemistry.

Preferred types of resins are Merrifield and Wang resins, Tentagel and PEG resin, optionally chemically modified to have a linker molecule between the functional group and the resin. The linker molecule facilitates the cleavage of the final molecule from the solid support. Such resins are known to the people skilled in the art of combinatorial chemistry as REM-resin, FMP-resin, FDMP-resin, HTP-resin, ICHO-resin, Ellman-resin, MBHA-resin, Rink Amide resin, Rink Acid resin, HMPB resin, Trityl chloride resin, HMBA resin etc.

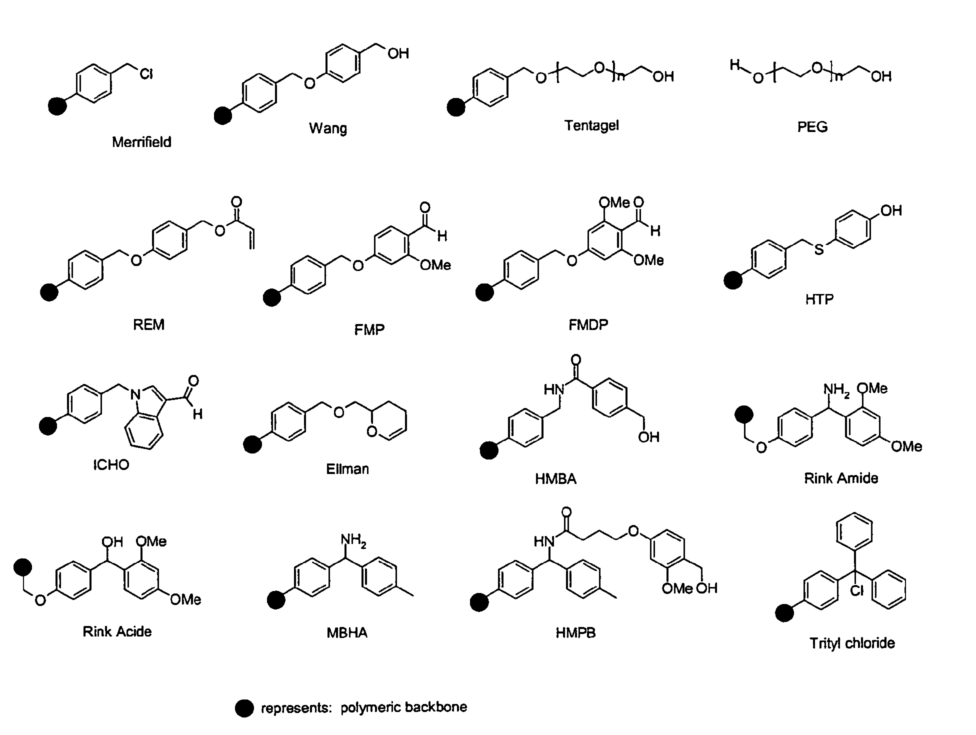

Particularly preferred are Merrifield and Wang resins, chemically modified to have a linker molecule to facilitate the cleavage, even more particularly preferred are REM-resins, FMP-resins and ICHO resins.

Such resins are commercially available, e.g. from Polymer Laboratories Inc., Amherst Fields Research Park, 160 Old Farm Road, Amherst MA 01002, U.S.A or from Novabiochem AG, c/o Merck Biosciences Ltd., Boulevard Industrial Park, Padge Road, Beeston, NOTTINGHAM, NG9 2JR, U.K.

The resin can be employed in any form, e.g. as a powder or in the form of solid objects, like Lanterns and Plugs. Preferred is their usage as powder in capsules.

In a preferred embodiment of the invention an ionic linker molecule is incorporated into the resin material. The ionic linker molecule connects the polymer matrix of the resin and the anchor group, to which the molecule that is being build up in the process of the library synthesis, is attached.

Examples of such ionic linkers include 3-alkyl-imidazolium-1-yl, 4-alkyl-pyridinium-1-yl, N,N-dialkyl-aza-alkandiyl. Preferred are linkers of the 3-alkyl-imidazolium-1-yl type.

In a preferred embodiment of the invention both the resin and the rf-tag are continued in a capsule that is permissible for dissolved reagents.

All kinds of such capsules that have been developed for use in combinatorial chemistry are suitable. Such capsules are typically made of polypropylene.

Suitable capsules are commercially available, e.g. from Discovery Partners Intl., 9640 Towne Centre Drive, San Diego CA 92121, U.S.A under the brand names MacroKan®, MiniKan® and MicroKan®.

In a further preferred embodiment of the invention the resin portions are formed into sizeable solid objects into which the rf-tag is embedded, e.g. the resin is shaped like a cylinder having a center hole into which the rf-tag is inserted.

Resins shaped into such objects are commercially available, e.g. from Mimotopes, San Diego, USA under the trade name SynPhase® Lanterns or from Polymer Laboratories Inc., Amherst, USA under the trade name StratoSphere Plugs®.

The microwave irradiation according to the invention can be carried out using commercially available microwave ovens, which are available e.g. from CEM Corporation, P.O Box 200, Matthews, NC 28106, U.S.A. under the brand names Discoverer Explorer® or Mars Synthesis® or from the company Milestone S.r.l., Via Fatebenefratelli, 1/5, 24010 Sorisole (BG), Italy under the brand name MLS Ethos®.

The microwave irradiation is generally in the range of 50-1000 W for 1-120 min.

The microwave irradiation can generally be used in any type of chemical reaction employed in the solid phase combinatorial synthesis of organic molecules that affords heating.

Such reaction include but are not limited to nucleophilic substitutions, radical substitutions, electrophilic aromatic substitutions, nucleophilic aromatic substitutions, cylizations, eliminations, additions to unsaturated bonds, oxidations, reductions, rearrangements, cleavage reactions, dehydratizations, organometallic reactions, acylations and reductive aminations.

Preferred types of chemical reactions are reductive aminations, acylations, transaminations, transesterifications, metathesis reactions, Aldol-reactions, cyclizations, cleavage off the solid support by cyclizations and cycloadditions.

Even more preferred types of chemical reactions are reductive aminations, acylations, transaminations, transesterifications and cleavage off the solid support by cyclizations.

In a further preferred embodiment of the invention the reaction where the microwave irradiation is employed is carried out in an autoclave. This allows reactions to be performed at temperatures beyond the boiling point of the solvent.

In addition, under microwave irradiation and increased temperatures, organic solvents as well as water experience changes in their dielectric constant (ε') and dielectric loss (ε"), meaning that the chemical nature of the solvent changes. Thus, this technique allows the extension of range of possible reaction conditions compared to classical approaches.

A further preferred embodiment of the invention is the addition of ionic liquids to the solvent system in which the reaction is carried out. Ionic liquids are salts, that at room temperature behave like an organic liquid although they are composed of anionic and cationic species and that are miscible with a wide range of organic solvents. Typical examples of ionic liquids are N-methyl-N'-butyl-imidazolium tetrafluoroborate 1 and N-methyl-N'-butyl-imidazolium hexafluorophosphate 2.

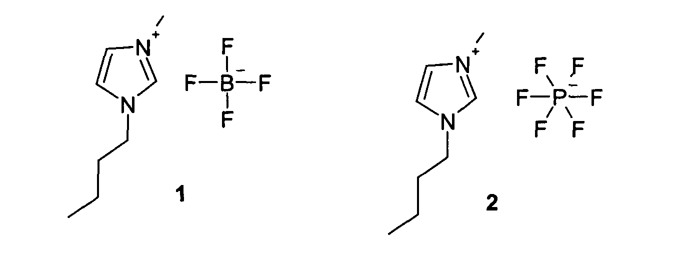

It was found that it is beneficial, to perform the desired transformations under microwave irradiation using mixture of ionic liquids and organic solvents. The vessels reached the reaction temperature more rapidly, the product yields were higher and thermal damages to the polymeric material of either resin or capsules were further minimized.

In carrying out the process according to the invention it is advantageous to immerse the resin or the capsule completely in the surrounding solvent in order to avoid degradation.

The invention is further illustrated but not limited by the following examples.

### General experimental details:

As a source for microwave heating, a commercially available CEM Discoverer-Explorer microwave oven was used. For irradiation of individual probes, a 10 mL test tube shaped glass vessel was charged with a capsule containing rf-tag and resin. The vessels were sealed with a teflon coated metallic lid, that were applied via pliers that created sealed reaction vessels. For irradiation of multiple capsules, an 80 mL thick walled glass insert was used that is being commercialized by CEM, that also can give a sealed vessel. The term "resin" refers to Merrifield resin that was N-alkylated with 3-formyl-indole to provide an acid labile linker and a formyl group as anchor group. Merrifield resin was purchased at Polymer Labs and had a loading of 1.3 mmol/g. Where mentioned, N-Methyl-N'-butyl-imidazolium tetrafluoroborate was added to the solvent system of reactions undergoing microwave heating.

### Comparative Example

Preparation of compound A (of table 1) under classical conditions: An IRORI MicroKan® was filled with 35 mg resin (0.0455 mmol) and a rf-tag. The resin was allowed to swell for 10 min in 1.2 mL CH₂Cl₂, before [NMe₄][BH(OAc)₃] (60 mg, 0.228 mmol) was added, together with a drop of MeOH and aniline (42 mg, 0.455 mmol). The reaction medium was vibrated for 48 h at r.t. At the end of that period, NaBH₃CN (14 mg, 0.228 mmol) was added as a solution in 0.1 mL MeOH. The reaction vessels was purged with N₂ and stirred for another 6 h at r.t.

The reaction was worked up by destroying excess of hydride by addition of small volumes of MeOH, filtration of the liquids and washing of the Kan with CH₂Cl₂, MeOH, CH₂Cl₂, MeOH, CH₂Cl₂, MeOH, CH₂Cl₂ (1.5 mL each washing). The Kan was dried overnight under vacuum.

The resin in the Kan was again allowed to swell in CH₂Cl₂,(1.2 mL), then pyridine (35 mg, 0.448 mmol) and 4-N-Dimethylamino-pyridine (11 mg, 0.09 mmol) was added and the reaction mixture vibrated for 10 min before Hex-5-in-carboxyl chloride was added under nitrogen atmosphere. The reaction was vibrated for 24 h at r.t., before all supernatant liquids were filtered off and the Kan washed with CH₂Cl₂, MeOH, CH₂Cl₂, MeOH, CH₂Cl₂, MeOH, CH₂Cl₂ (1.5 mL each washing).

For the cleavage, the Kan was shaken for 3 h in a solvent mixture of CH₂Cl₂ and F₃CCO₂H (9:1), (1.5 mL). After that time, the sample was filtered. The Kan was washed with MeCN (1 mL) and the mixture again filtered. Both filter solutions were combined and evaporated to give the product. The product was weighed and characterized by LC-MS and ¹H-NMR.

### Example

Preparation of compound C with microwave irradiation: An IRORI MicroKan® was filled with 35mg resin (0.0455 mmol) and a rf-tag. The resin was allowed to swell for 10 min in 1.2 mL THF, then 2,4,6-Trichloroaniline was added (89 mg, 0.455 mmol) and N-Butyl-N'-methyl-imidazolium tetrafluoroborat (0.1 mL) und 11 mL HOAC. After a period of 5 min [NMe₄][BH(OAc)₃] (120 mg, 0.455 mmol) was added as a solution in 0.4 mL DMF. By using microwave irradiation (150 W, 20 bar) the vessel was heated to 70°C for 60 min. Soon after the end of heating, the reaction was worked up by destroying excess of hydride by addition of small volumes of MeOH, then the liquids were filtered off and the Kan washed with MeOH, CH₂Cl₂, CH₂Cl₂, MeOH, CH₂Cl₂, MeOH, CH₂Cl₂ . Where the Kan was covered with a white coating, the Kan was washed with H₂O: HOAc (9:1, 1.5 mL, 3x), then H₂O: THF (1:3, 1.5 mL, 7x). The Kan was dried overnight under vacuum.

The resin in the Kan was again allowed to swell in CH₂Cl₂,(1.2 mL), then pyridine (35 mg, 0.448 mmol) and 4-N-Dimethylamino-pyridine (11 mg, 0.09 mmol) was added and the reaction mixture vibrated for 10 min before Hex-5-in-carboxyl chloride was added under nitrogen atmosphere. The reaction was vibrated for 24 h at r.t., before all supernatant liquids were filtered off and the Kan washed with CH₂Cl₂, MeOH, CH₂Cl₂, MeOH, CH₂Cl₂, MeOH, CH₂Cl₂ (1.5 mL each washing).

For the cleavage, the Kan was shaken for 3 h in a solvent mixture of CH₂Cl₂ and F₃CCO₂H (9:1), (1.5 mL). After that time, the sample was filtered. The Kan was washed with MeCN (1 mL) and the mixture again filtered. Both filter solutions were combined and evaporated to give the product. The product was weighed and characterized by LC-MS and 1-H-NMR.

According to these procedures the following compounds were obtained:

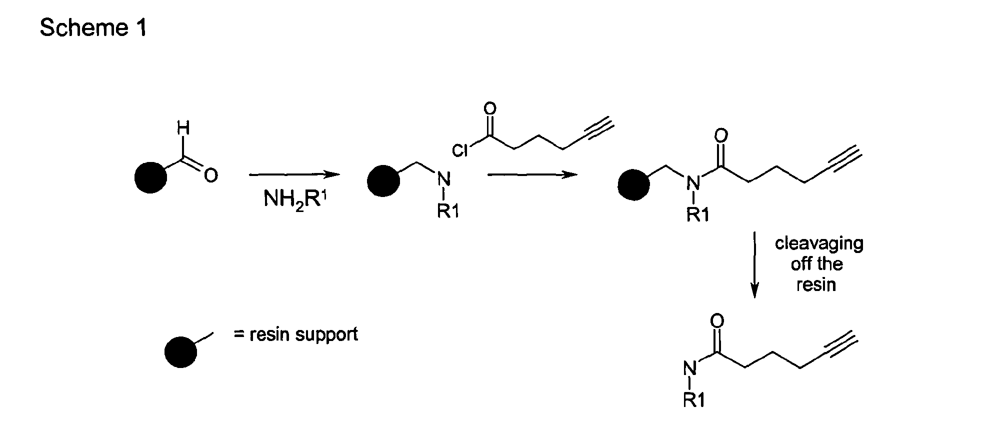

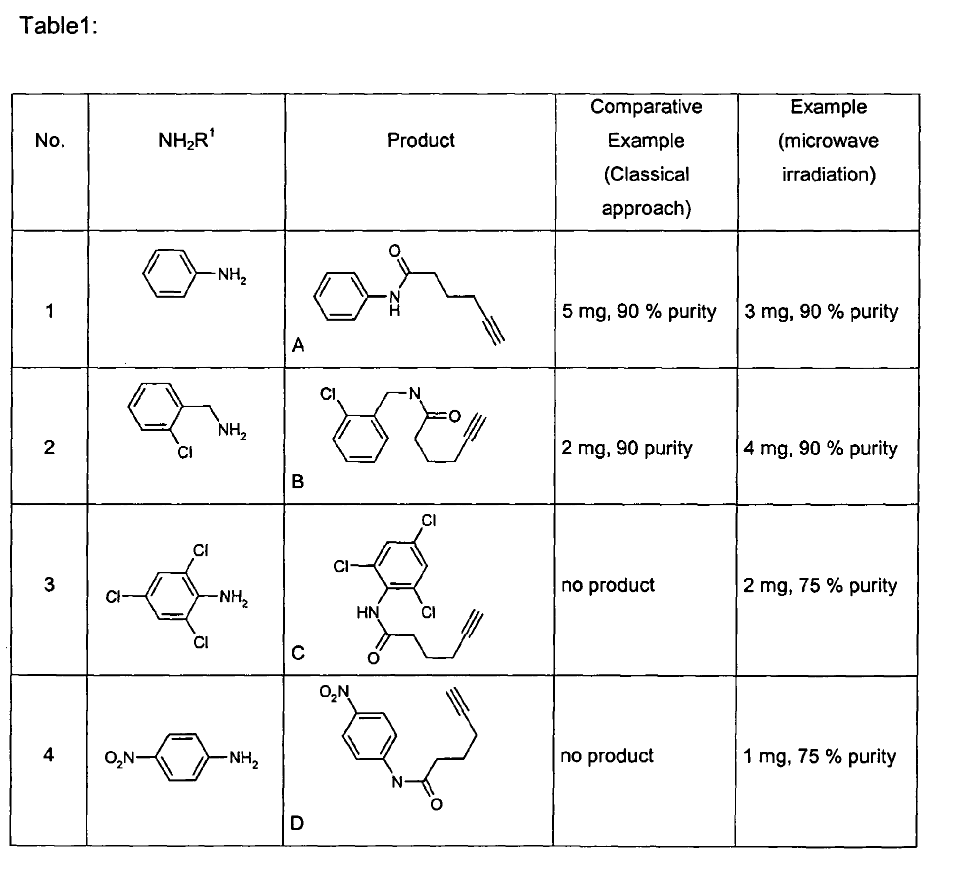

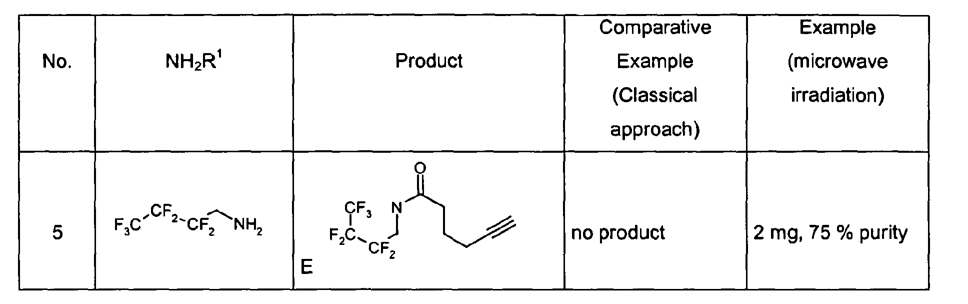

As can be seen in Table 1, in the absence of microwave irradiation it was not possible to obtain the desired series of transformation with electron deficient amines or anilines like 2,4,6-Trifluoroaniline, 4-Nitroaniline or 2,2,3,3,4,4,4-Heptafluorobutylamine. In contrast, under microwave conditions the desired amide products were isolated in purity of 75 % (C, D and E).

## Claims

1. A process for the production of a combinatorial library of where a plurality of organic molecules is synthesized by chemically fixing a plurality of starting materials each to a separate portion of a solid support, which is combined with a radiofrequency tag, building up the organic molecules in multiple reaction steps, and finally cleaving of the desired organic molecules, where at least one of the multiple reaction steps is carried out under microwave irradiation.

2. The process as claimed in claim 1, where a ionic linker group is incorporated into the solid support, connecting a polymeric matrix and an anchor group to which the synthesized organic molecule is attached.

3. The process as claimed in claim 1 or 2, where an ionic liquid is added to a solvent system in which the reaction step under microwave irradiation is taking place.

4. The use of microwave irradiation in the synthesis of combinatorial libraries on solid supports that are combined with radiofrequency tags.

5. A device for the production of a combinatorial library comprising a microwave oven with means for orbitally or linearly shaking vessels inside the microwave cavity.
